# EUROPÄISCHE PATENTSCHRIFT

(11) **EP 0 580 685 B1**
(45) Veröffentlichungstag und Bekanntmachung des Hinweises auf die Patenterteilung: **03.01.1996**
(21) Anmeldenummer: 92908637.9
(22) Anmeldetag: 15.04.1992
(51) Int. Cl.: A61K 38/46

(54) **PHARMAZEUTISCHE VERPACKUNGSEINHEIT ENTHALTEND PLASMINOGENAKTIVATOREN ZUR MEHRFACHBOLUSGABE**
PHARMACEUTICAL PACKAGING UNIT CONTAINING PLASMINOGEN ACTIVATORS FOR MULTIPLE-BOLUS ADMINISTRATION
UNITE DE CONDITIONNEMENT PHARMACEUTIQUE CONTENANT DES ACTIVATEURS DE PLASMINOGENE DESTINES A ETRE ADMINISTRES EN PLUSIEURS BOLS

(30) Priorität: 16.04.1991 DE 4112398; 18.07.1991 DE 4123845
(43) Veröffentlichungstag der Anmeldung: 02.02.1994
(60) Teilanmeldung: 95106049.0
(73) Patentinhaber: BOEHRINGER MANNHEIM GMBH, D-68298 Mannheim (DE)
(72) Erfinder: MARTIN, Ulrich, D-68298 Mannheim (DE); KOENIG, Reinhard, D-68298 Mannheim (DE)
(74) Vertreter: Mink, Reinhold, Dr.
(86) Internationale Anmeldenummer: EP9200851
(87) Internationale Veröffentlichungsnummer: WO9218157

(56) Entgegenhaltungen:
- EP-A- 0 211 592
- EP-A- 0 382 174
- WO-A-90/08557
- WO-A-91/08765
- "Dictionnaire Vidal", 66. Auflage, 1990, Seiten 13-14, Editions Du Vidal, Paris, FR, siehe Seiten 13-14: "Actilyse"
- Coronary Artery Disease, Band 1, Nr, 1, Januar/Februar 1990, B. TRANCHESI et al.: "Intravenous bolus administration of recombinant tissue plasminogen activator to patients with acute myocardial infarction", Seiten 83-88, siehe das ganze Dokument (in der Anmeldung erwähnt)

## Beschreibung

Gegenstand der vorliegenden Erfindung ist eine pharmazeutische Verpackungseinheit enthaltend Plasminogenaktivatoren zur Mehrfachbolusgabe, wobei der Plasminogenaktivator in zwei oder mehreren getrennten Behältnissen vorliegt.

Die thrombolytische Therapie des Herzinfarktes ist eine effektive, medizinisch gut erprobte und belegte Therapie zur Beseitigung von Verschlußthromben in den Koronararterien des Herzens. Das gentechnologisch hergestellte t-PA (tissue-type plasminogen activator) ist zur Therapie dieser Erkrankung weltweit im Einsatz und hat sich als effektiv zur Auflösung koronarer Thromben erwiesen (Verstraete et al., Lancet II 1985; 965-969). Durch frühzeitige Lysetherapie des Herzinfarktes kann die residuale Herzfunktion nach Herzinfarkt im Vergleich zu anderen Therapien verbessert werden (Armstrong et al., J. Am. Coll. Cardiol. 1989; 13: 1469-1476) und eine höhere Überlebensrate gegenüber anderen Therapien erzielt werden (Wilcox et al., Lancet II, 1988; 525-539).

Große Thrombolysestudien mit insgesamt mehreren tausend Patienten zeigen, daß eine rasche Einleitung der Thrombolyse mit frühzeitiger Reperfusion des Myokardgewebes zu einer Rettung von Myokardgewebe und damit zu einem Anstieg der Überlebensrate führt (GISSI-study-group, Lancet I, 1986; 397-401). Aus diesem Grunde ist es notwendig, die thrombolytische Therapie zu einem Zeitpunkt einzuleiten, zu dem das hinter dem Koronarverschluß gelegene Gewebe noch nicht irreversibel geschädigt ist.

Prinzipiell besteht bei der Behandlung eines Koronarverschlusses das Problem, einen möglicherweise auftretenden Wiederverschluß des Infarktgefäßes nach erfolgreicher initialer Thrombolyse zu vermeiden. Für t-PA (Alteplase) konnte dieser nachteilige Effekt beobachtet werden (Chesebro et al., Circulation 1987; 76: 142-154). Der Wiederverschluß des Infarktgefäßes führt zu erhöhter Morbidität und Mortalität. Zur Verhinderung von Wiederverschlüssen werden Substanzen verschiedener pharmakologischer Wirkprinzipien, wie Heparin (Bleich et al., Am. J. Cardiol. 1990; 66: 1412-1417) und Acetylsalicylsäure (Hsia et al., N. Engl. J. Med. 1990; 323: 1433-1437) eingesetzt. Auch die zeitlich verlängerte Infusion von t-PA (Alteplase) soll durch eine längere Thrombolysedauer den Wiederverschluß des Infarktgefäßes verhindern (Gold et al., Circulation 1986; 73: 347-352; Verstraete et al., Am. J. Cardiol. 1987; 60: 231-237; Johns et al., Circulation 1988; 78: 546-556). Nach Beendigung der Infusion wird allerdings häufig das Auftreten von Reokklusionen beobachtet.

Rekombinant hergestelltes t-PA (rt-PA) wurde bereits im Rahmen von klinischen Voruntersuchungen an wenigen Patienten als Doppel- oder Mehrfachbolus verabreicht. Zwar wurden kurzfristig, d.h. bis 90 Minuten nach der ersten Bolusgabe, hohe Auflösungsraten der Thromben gefunden, jedoch wurde aufgrund der hohen Dosen eine extensive, systemische Plasminogen-Aktivierung mit nachfolgender, fast völliger Reduzierung des Fibrinogens (nur noch 15.5 bis 5.2 % des Ausgangswertes) beobachtet (J. Am. Coll. Cardiol. 1991, 17(2), 152A). Diese unerwünschte Reduzierung des Fibrinogenspiegels stellt insofern einen Nachteil dar, als bei Notoperationen eine hohe Blutungsneigung besteht, und eine intensive Überwachung des Patienten notwendig ist. Ferner wurde bei Doppel- oder Dreifachbolusgabe von rt-PA nachteilig eine Reokklusionsneigung, d.h. ein Wiederverschluß des Gefäßes, beobachtet (Circulation 1990, 82(4), Suppl. III 538, abstract 2137; Br. J. Haematol., 1991, 77, Suppl. 1, 47, abstract P080). Für Alteplase wurde ebenfalls eine Studie durchgeführt, in der die Mehrfachbolusgabe näher untersucht wurde (Coronary Artery Desease, 1990, 1(1), 83-88).

Aufgrund der oben genannten nachteiligen Effekte hat die Applikation von rt-PA in Form eines Doppel- oder Mehrfachbolus keine praktische Anwendung gefunden. Darüberhinaus ist die Bolusgabe von rt-PA schon allein deshalb für unzweckmäßig angesehen worden, da die Halbwertszeit von rt-PA relativ kurz ist und nur 3-6 Minuten beträgt (Garabedian et al., J. Am. Coll. Cardiol. 1987; 9: 599-607). Dies bedeutet, daß zur Sicherstellung des Thrombolyseerfolgs eine relativ langdauernde Infusion zur Aufrechterhaltung effektiver Plasmaspiegel erforderlich ist. In Notfallsituationen stellt jedoch diese längerdauernde Infusion der Substanz (30 Minuten bis 6 Stunden) deutliche Handling-Nachteile dar. Außerdem besteht dann eine erhöhte Gefahr des Auftretens von Blutungen (Marder and Sherry, N. Engl. J. Med. 1988; 318: 1512-1520).

Es wurde nun überraschenderweise gefunden, daß Proteine mit plasminogenaktivator-ähnlicher Wirkung und einer gegenüber t-PA verlängerten Halbwertszeit erfolgreich bei der Behandlung von Koronarverschlüssen eingesetzt werden können, wenn die eingesetzten Proteine in Form einer fraktionierten Gabe von zwei oder mehreren Bolusinjektionen appliziert werden. Auf diese Weise wird eine schnelle und einfache Verabreichung von Proteinen mit plasminogenaktivator-ähnlichen Eigenschaften zur effektiven Behandlung von thromboembolischen Erkrankungen ermöglicht.

Der Begriff "fraktionlerte Gabe" bedeutet im Sinne der vorliegenden Erfindung die Applikation einer therapeutisch wirksamen Menge eines Plasminogenaktivators in zwei oder mehreren Teilmengen in Form eines Doppel- oder Mehrfachbolus.

Die Bolusgabe ist eine intravenöse Schnellinjektion und insbesondere deshalb vorteilhaft, weil einerseits die Zeit vom Beginn der klinischen Symptomatik bis zur Behandlung, und andererseits auch die Zeit vom Behandlungsbeginn bis zur Auflösung des Koronarthrombus verkürzt wird und damit mehr Myokardgewebe vor einer irreversiblen Zerstörung gerettet werden kann. Die erfindungsgemäße Doppel- oder Mehrfachbolusgabe bewirkt eine höhere thrombolytische Potenz der verwendeten Plasminogenaktivatoren. Dadurch ist nicht nur eine Dosisverringerung im Vergleich zu Alteplase möglich, sondern es findet sich überraschenderweise nach der Bolusinjektion eine schnellere Reperfusion als nach entsprechender Gabe in Form einer Infusion. Außerdem bewirkt die Doppel- oder Mehrfachbolusgabe eine signifikante Verlängerung der kumulativen Patencyzeit (Summe der Zeitintervalle nach Reperfusion, in denen ein koronarer Blutfluß vorhanden ist), sowie eine deutliche Erhöhung des koronaren Blutflusses, wobei dieser auch noch längere Zeit nach der Applikation auf einem relativ hohen Niveau stabil bleibt. Ferner hat die Doppel- oder Mehrfachbolusinjektion den überraschenden Vorteil eines geringeren Abfalls des Plasmafibrinogens im Gegensatz zur Einmalbolusinjektion. Diese vorteilhaften Eigenschaften einer Zwei- oder Mehrfachbolusgabe von Plasminogenaktivatoren konnten allerdings nicht erreicht werden, wenn die Gabe der entsprechenden Gesamtmenge des Plasminogenaktivators durch eine einmalige Bolusinjektion erfolgte.

Thromboembolische Erkrankungen im Sinne der vorliegenden Erfindung sind insbesondere solche Erkrankungen, die auf einen Herzinfarkt oder auf eine Reokklusion der Koronararterien zurückzuführen sind, wobei Reokklusionen vor allem bei der Anwendung von Thrombolytica zur Behandlung des Herzinfarktes auftreten. Ursache für einen Herzinfarkt ist die Entstehung eines Thrombus in den Koronararterien. Dieser Thrombus besteht aus einer Kombination von Fibrin und Thrombozyten. Primäres Ziel bei der Behandlung des Herzinfarktes ist die rasche Auflösung dieses Thrombus und die Wiederherstellung des Blutflusses (Reperfusion). Eine erfolgreiche Therapie muß den Thrombus möglichst schnell auflösen und seine Wiederbildung (Reokklusion) verhindern. Besonders vorteilhaft eignet sich die erfindungsgemäße Doppel- oder Mehrfachbolusgabe zur Behandlung des Hirnschlages.

Die erfindungsgemäße pharmazeutische Verpackungseinheit besteht aus einer Darreichungsform, die den Plasminogenaktivator in zwei oder mehreren getrennten Behältnissen enthält. Ferner können die Verpackungseinheiten einen Hinweis enthalten, beispielsweise in Form eines für Arzneimittel vorgeschriebenen Beipackzettels, aus dem hervorgeht, daß die Applikation einer therapeutisch wirksamen Menge des Plasminogenaktivators vorteilhafterweise durch Doppel- oder Mehrfachbolusgabe erfolgt. Diese Information über die Art und Weise der Anwendung kann auch als Verpackungsaufdruck auf dem verkaufsfertigen Arzneimittelpräparat angegeben werden, bzw. aus einem Informationsblatt entnommen werden, das mit Arzneimittelpräparten, die Plasminogenaktivatoren im Sinne der vorliegenden Erfindung enthalten, in Zusammenhang gebracht werden kann.

Als geeignete Darreichungsform für Plasminogenaktivatoren kommen aus dem Stand der Technik bekannte galenische Formulierungen, beispielsweise Lyophilisate oder Lösungen in entsprechenden Behältnissen, wie z. B. in Ampullen, in Frage. Diese Formulierungen enthalten in der Regel übliche pharmazeutische Hilfsstoffe, die zur Herstellung von isotonischen Lösungen geeignet sind, und gegebenenfalls weitere Stabilisierungs- oder Solubilisierungsmittel.

Als Proteine mit plasminogenaktivator-ahnlicher Wirkung (im Sinne der vorliegenden Erfindung auch kurz "Plasminogenaktivatoren" genannt) werden solche Proteine eingesetzt, die gegenüber t-PA eine verlängerte Halbwertszeit besitzen. Plasminogenaktivatoren bewirken die Auflösung von Thromben und ermöglichen dadurch, daß wieder Blut durch die betreffenden Blutgefäße fließt. Wie oben bereits erwähnt beträgt die Halbwertszeit von t-PA im menschlichen Plasma ungefähr 3-6 Minuten. Bevorzugt im Sinne der vorliegenden Erfindung kommen solche Plasminogenaktivatoren in Frage, die eine gegenüber dem t-PA mindestens um den Faktor 2, vorzugsweise 3-7 und insbesondere 3-5 verlängerte Halbwertszeit besitzen. Insbesondere sollte die Halbwertszeit mindestens etwa 10 Minuten, beispielsweise 10-90, 10-40 oder 10-20 Minuten betragen. Bevorzugt werden solche Proteine verwendet, deren Halbwertszeit um den Faktor 3-30, insbesondere 5-20 mal länger ist als die Halbwertszeit von t-PA.

Im Sinne der vorliegenden Erfindung kommen als Proteine mit plaminogenaktivator-ähnlicher Wirkung beispielhaft die folgenden Plasminogen-Aktivatoren in Frage: LY 210825 (K2P aus "syrian hamster cell line", Circ. 1990, 82, 930-940); DeltaFE3x und DeltaFE1x (K1K2P aus "chinese hamster ovary cells", Blood 1988, 71, 216-219); DeltaFEK1 (K2P aus "mouse C127 cells", J. Cardiovasc. Pharmacol. 1990, 16, 197-203); E-6010 (Jap. Circ. J. 1989, 53, p. 918); t-PA-Varianten (Thromb. Haemost. 1989, 62, p. 542); K2P und D-K2P (Thromb. Haemost. 1989, 62, p. 393); MB-1018 (FK2K2P, Thromb. Haemost. 1989, 62, p. 543); FK2P (FASEB J. 1989, 3, A1031, abstract 4791); Delta1x (Circulation 1988, 4, II-15); K1K2P (Thromb. Res. 1988, 50, 33-41); FK1K2P (J. Biol. Chem. 1988, 263, 1599-1602). Insbesondere werden rekombinante Plasminogen-Aktivatoren vom Typ K2P, wie z.B. BM 06.022 (bekannt aus EP-A-0 382 174) eingesetzt. Weitere t-PA-Muteine dieser Art sind in den folgenden Patentanmeldungen beschrieben: EP-A-0,196,920, EP-A-0,207,589; AU 61804/86; EP-A-0,231,624; EP-A-0,289,508; JP 63133988; EP-A-0,234,051; EP-A-0,263,172; EP-A-0,241,208; EP-A-0,292,009; EP-A-0,297,066; EP-A-0,302,456; EP-A-0,379,890.

Das pharmakokinetische Profil von rt-PA (Alteplase) ist typischerweise durch ein Zwei- oder sogar Dreikompartimentmodell charakterisiert (Thromb. Haemost. 1989, 61, 497-501). Am wichtigsten ist dabei die initiale Halbwertszeit, da sie mit 66 % zur Gesamtfläche unter der Kurve (area under the curve = AUC) beitragt. Daher wird diese Halbwertszeit als die dominante Halbwertszeit bezeichnet. Bei von t-PA abgeleiteten Plasminogen-Aktivatoren mit verlängerter Halbwertszeit ist naturgemäß auch das pharmakokinetische Profil verändert. Die Bezeichnung "verlängerte Halbwertszeit", bezieht sich dabei auf die dominante Halbwertszeit, die charakteristisch für das pharmakokinetische Profil ist, weil sie quantitativ am meisten zur Gesamt-AUC beiträgt. Die Bestimmung der Halbwertszeit erfolgt nach dem aus dem Stand der Technik bekannten Verfahren (Pharmocokinetics, Ch. 2, Marcel Dekker, New York 1982).

Als Plasminogenaktivatoren kommen prinzipiell solche, insbesondere rekombinant hergestellte Derivate des t-PA in Frage, die im wesentlichen diejenigen Proteinbereiche des natürlichen Proteins umfassen, die für die Fibrinolyse der Thromben zuständig sind. Dabei können auch solche Derivate des t-PA verwendet werden, die Deletionen oder Substitutionen einzelner oder mehrerer Aminosäuren in der Sequenz des t-PA aufweisen, solange die Halbwertszeit solcher Derivate gegenüber t-PA im obigen Sinne verlängert ist. Insbesondere sind dies t-PA-Derivate vom Typ K2P.

Beispielhaft für die im Sinne der vorliegenden Erfindung in Frage kommenden Plasminogenaktivatoren wurde der in der Europäischen Patentanmeldung EP-A-0,382,174 näher beschriebene Plasminogenaktivator K2P (BM 06.002) verwendet. Er besteht aus den Kringel 2 (K) und Protease-(P)-Domänen des humanen t-PA und liegt wegen seiner Expression in Escherichia-Coli-Zellen in unglycosilierter Form vor. Die spezifische Aktivität von K2P beträgt 550.000 +/- IU/mg (= 550 +/- 200 KU/mg). K2P wird zur thrombolytischen Therapie thrombembolischer Krankheiten (Herzinfarkt, Lungenembolie, Hirnschlag und andere gefäßverschließende Erkrankungen) eingesetzt.

Die fehlende Glycosilierung führt zu einer herabgesetzten Clearance und resultiert in einer gegenüber dem t-PA um mindestens den Faktor 3 - 4 verlängerten Halbwertszeit. Die verlängerte Halbwertszeit macht die Anwendung der Substanz als klinisch wünschenswerte Mehrfach-Bolusapplikation möglich. Der thrombolytische Effekt der Bolusgabe der Substanz wurde in verschiedenen Tiermodellen untersucht. Dabei zeigte sich eine ungefähr um den Faktor 5 erhöhte thrombolytische Wirksamkeit verglichen mit Alteplase. Besonders beeindruckend war dabei die im Vergleich zu Alteplase und anderen Thrombolytika rasche Eröffnung von thrombotisch verschlossenen Gefäßen, was eine gegenüber anderen Thrombolytika verminderte Zeit zur Gefäßeröffnung und damit eine raschere Wiederdurchblutung des infarzierten Myocardgewebes bedeutet.

Aufgrund der verbesserten pharmakokinetischen Eigenschaften von K2P kann die effektive zu applizierende Dosis verringert und K2P kann als i. v. Mehrfachbolusinjektlon verabreicht werden. Damit wird eine sehr schnelle maximale Reperfusionsrate erreicht. Weiterhin kann durch die Verabreichung eines Doppel- oder Mehrfachbolus die Durchblutungsverschlechterung in der Koronararterie nach Reperfusion (Reokklusionsneigung) signifikant verringert oder verhindert werden. Zusätzlich ist nach Doppel- oder Mehrfachboli der Abfall des Plasmafibrinogens geringer als nach der Einmalbolusinjektion der gleichen Gesamtdosis.

Neben der raschen Eröffnung von thrombotisch verschlossenen Gefäßen ist die Verhinderung des Wiederverschlußes der eröffneten Gefäße von großer klinischer Bedeutung, da ein erneuter Verschluß des Infarktgefäßes zu einem klinisch bedeutsamen Reinfarkt mit den daraus resultierenden klinischen Komplikationen führen kann.

Die Applikation von K2P als Bolus in äquieffektiver Dosis im Vergleich zur Infusion von Alteplase führt im Tiermodell zu einer überraschend schnellen Eröffnung der Infarktgefäße und ermöglicht damit eine schnelle Durchblutung des infarzierten Myocardgewebes. Im Lichte der gegenwärtigen Kenntnisse über die Thrombolyse ist dieser beobachtete Effekt aus klinischer Sicht als sehr positiv zu werten.

Das Problem des Wiederverschlusses von thrombolysierten Infarktgefäßen tritt nach den tierexperimentellen Daten auch unter dem Einmal-Bolus von K2P in einem Maße auf, das mit anderen Thrombolytika vergleichbar ist. Überraschenderweise zeigt sich, daß eine Aufteilung der Gesamtdosis an K2P in zwei oder mehrere zeitlich versetzt injizierte Boli das Auftreten von Durchblutungsverschlechterungen in der Koronararterie nach Reperfusion verringert bzw. sogar verhindert.

Der zeitliche Abstand zwischen Beginn der ersten und einer darauffolgenden Bolusinjektion ist indikationsabhängig und kann zwischen 10 Minuten und 6 Stunden, vorzugsweise 20 Minuten und 2 - 3 Stunden, insbesondere 30 - 90 Minuten betragen. Die Injektionsdauer an sich ist relativ kurz und beträgt in Abhängigkeit von dem zu applizierenden Volumen etwa 0,5 - 3 Minuten, wobei eine Injektionsrate von etwa 5 - 10 ml/min. vorteilhaft ist. Im Sinne der vorliegenden Erfindung versteht man unter dem Begriff Mehrfachbolusgabe auch die kumulierte Applikation von zwei oder mehreren Einzelbolusinjektionen, die im Rahmen einer Intervalltherapie verabreicht werden. Dabei werden zwei oder mehrere, vorzugsweise drei, Bolusinjektionen täglich vorgenommen, wobei sich die Therapie über mehrere aufeinarderfolgende Tage hinweg erstrecken kann. Gegebenenfalls kann dieses Behandlungsschema auch durch ein oder zwei Tage unterbrochen werden, so daß die Applikation in Form von Bolusinjektionen beispielsweise an jedem zweiten Tag während der Gesamtbehandlungsdauer erfolgt. Von der bekannten Einfachbolusgabe unterscheidet sich diese kumulierte Gabe von Einfachboli im wesentlichen dadurch, daß die Applikation täglich mehrmals wiederholt wird. Ist der Zeitabstand zwischen der ersten und einer darauffolgenden Bolusinjektion relativ kurz, etwa 10 Minuten bis eine Stunde, spricht man von einer Doppelbolus- bzw. Mehrfachbolusgabe. Sind die Zeitabstände in der Größenordnung von mehreren Stunden, so kann auch von einer kumulierten Gabe von Einzelboli gesprochen werden.

Zur Herstellung der pharmazeutischen Darreichungsform bezüglich des Proteins mit plasminogenaktivator-ähnlicher Wirkung werden die üblichen pharmazeutischen Hilfs- oder Zusatzstoffe verwendet. Ferner können Stabilisierungs- oder die bekannten Solübilisierungsmittel, wie z.B. basische Aminosäuren (Arginin, Lysin oder Ornithin) zugesetzt werden. Geeignete galenische Darreichungsformen sind aus dem Stand der Technik bekannt oder können nach den allgemein üblichen Methoden hergestellt werden (vgl. EP 0,217,379; EP 0,228,862; WO 91/08763; WO 91/08764; WO 91/08765; WO 91/08766; WO 91/08767 oder WO 90/01334). Die Darreichungsform kann in lyophilisierter Form oder als gebrauchsfertige Injektionslösung vorliegen. Die Bolusinjektion kann intravenös, intramuskulär oder auch subcutan erfolgen, wobei die i.v.-Injektion bevorzugt ist.

Bei der Herstellung der pharmazeutischen Verpackungseinheit können die zu verabreichenden Mengen des Proteins für die Zwei- oder Mehrfachbolusinjektion in einem oder mehreren, vorzugsweise in zwei getrennten Behältnissen, wie z. B. in Ampullen, zur Verfügung gestellt werden. Es ist auch möglich, eine Multidose-Darreichungsform zur Verfügung zu stellen, aus der die jeweils gewünschte Menge des Proteins durch Abziehen auf eine geeignete Spritze entnommen werden kann. Die Ampullen können die bereits injektionsfertige Lösung oder alternativ ein Lyophilisat des Proteins, und gegebenenfalls weitere pharmazeutisch übliche Hilfs- oder Trägerstoffe enthalten. Die Lyophilisate werden mit üblichen Injektionslösungen kurz vor der Applikation versetzt, so daß eine Lösung entsteht, die direkt appliziert werden kann.

Die Menge des Proteins mit plasminogenaktivator-ähnlicher Wirkung kann in dem ersten und zweiten Behältnis in Abhängigkeit der jeweiligen Bedürfnisse gleich oder auch verschieden sein. In der Regel werden Mengen von je 3-50 MU pro Behältnis verwendet. Bevorzugt wird für die erste Bolusinjektion eine größere Menge des Proteins verabreicht als für die zweite Bolusinjektion. Insbesondere werden Mengen von 5-20 MU, insbesondere 5-15 MU für die erste und 3-15 MU, insbesondere 3-10 MU für die Zweite Bolusinjektion verwendet. Besonders bevorzugt verwendet man etwa 10 MU für die erste und etwa 5 MU für die zweite Injektion. Die kumulative Dosis liegt bevorzugt im Bereich von 15-40 MU.

Anhand der folgenden Beispiele wird die Erfindung näher erläutert:

### Beispiel 1

Für die Versuche wurde ein Hundemodell zur Simulation des Herzinfarktes benutzt. Erwachsene Beaglehunde beiderlei Geschlechts wurden mit Barbiturat narkotisiert, intubiert und künstlich beatmet. Es wurden arterielle und venöse Katheter gelegt, um den Blutdruck zu kontrollieren, Substanzen zu verabreichen oder Blutproben zu entnehmen. Der Brustkorb wurde eröffnet und das Herz wurde dargestellt. Ein kurzer Abschnitt des Ramus circumflexus der Arteria coronaria sinistra wurde isoliert und präpariert. Anschließend wurde die Koronararterie mit folgenden Instrumenten von proximal nach distal versehen: Einem elektromagnetischen Kopf zur Messung des Blutflußes in der Herzkranzarterie, eine Stimulationselektrode, eine einstellbare Schraube und einen Faden. Die Spitze der Stimulationselektrode wurde durch die Wand der Herzkranzarterie durchgeführt und innerhalb des Gefäßes so gelegt, daß die Nadelspitze mit der Innenfläche des Gefäßes in Berührung kam. Die Schraube wurde so eingestellt, daß 90 % der reaktiven Hyperämie in Folge einer kurzfristigen Durchblutungsunterbrechung der Koronararterie ausgeschaltet wurden.

Der Thrombus in der Koronararterie zur Auslösung des Herzinfarktes wurde nach der ursprünglich von Romson et al. (Thromb. Res. 1980; 17: 841-853) beschriebenen Methode gebildet. Die Methode wurde in der modifizierten Fassung von Shebuski et al. (J. Pharmacol. Exp. Ther.1988; 246: 790-796) angewendet. Über die Stimulationselektrode wurde ein 150 mikro Ampere Gleichstrom an die Koronararterie angelegt und so lange aufrechterhalten bis der Blutfluß in der Koronararterie auf 0 ml/min abfiel und mindestens 3 Minuten dort blieb. Anschließend durfte der Thrombus noch 30 Minuten altern. In dieser Zeit wurden die Tiere heparinisiert in einer Dosis von 1000 units/Tier/Stunde. 30 Minuten nach Thrombusbildung wurde das Fibrinolytikum oder das Lösungsmittel als eine einminütige i. v. Bolusinjektion sechs Tieren pro Gruppe verabreicht. BM 06.022 wurde in vier Dosen verabreicht: 50, 100, 140 und 200 KU/kg. Alteplase wurde ebenfalls in vier Dosen gegeben: 200, 800, 1130 und 1600 KU/kg (= 2mg/kg). Die spezifische Aktivität von Alteplase betrug 800.000 IU/mg (= 800 kU/mg). Plasmaproben wurden vor und wiederholt nach Injektion der Fibrinolytika gewonnen, um die Konzentration der Aktivität von BM 06.022 oder Alteplase nach der Methode von Verheijen et al. (Thromb. Haemostas. 1982; 48: 266-269) zu bestimmen. Eine Reperfusion wurde angenommen, falls mindestens 33 % des Ausgangsblutflußes in der Koronararterie wiedererreicht wurden. Entsprechend wurde die Zeit bis zur Reperfusion vom Beginn der Injektion bis zum Erreichen dieses Blutflußes definiert. In je einer weiteren Versuchsgruppe wurde BM 06.022 in einer Dosis von 140 KU/kg und Alteplase in einer Dosis von 800 KU/kg (= 1 mg/kg) intravenös über 90 Minuten als Dauerinfusion verabreicht (10 % der Gesamtdosis als initialer i. v. Bolus).

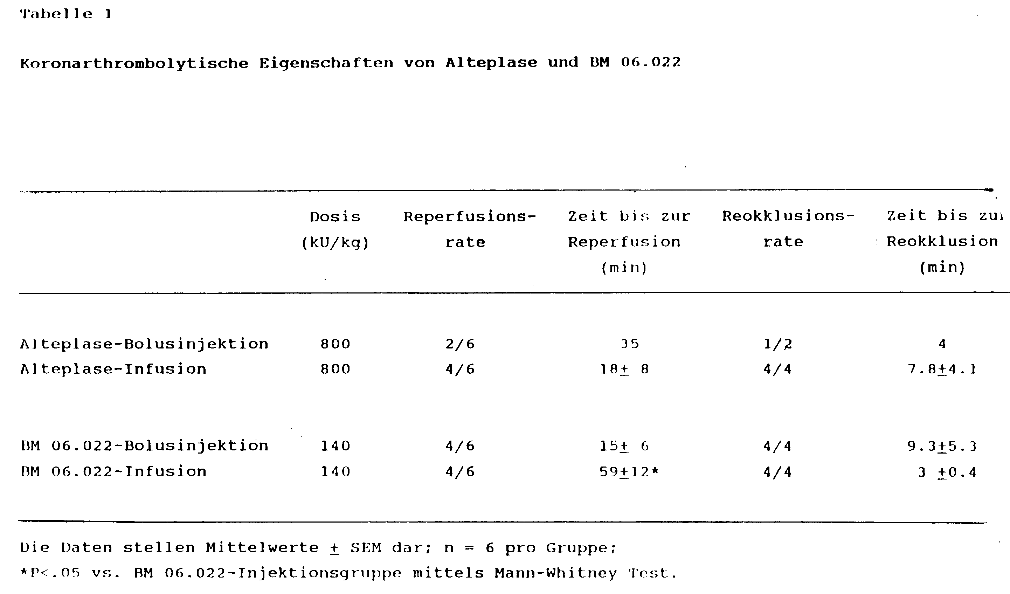

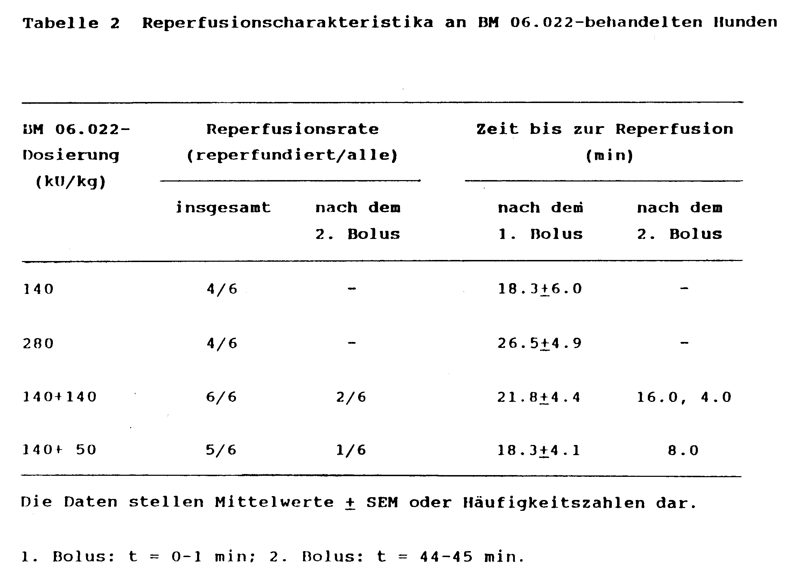

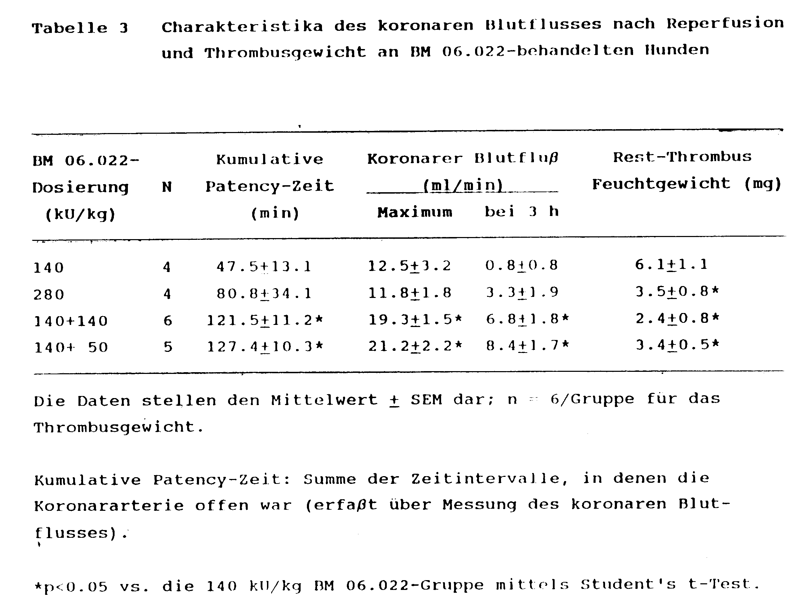

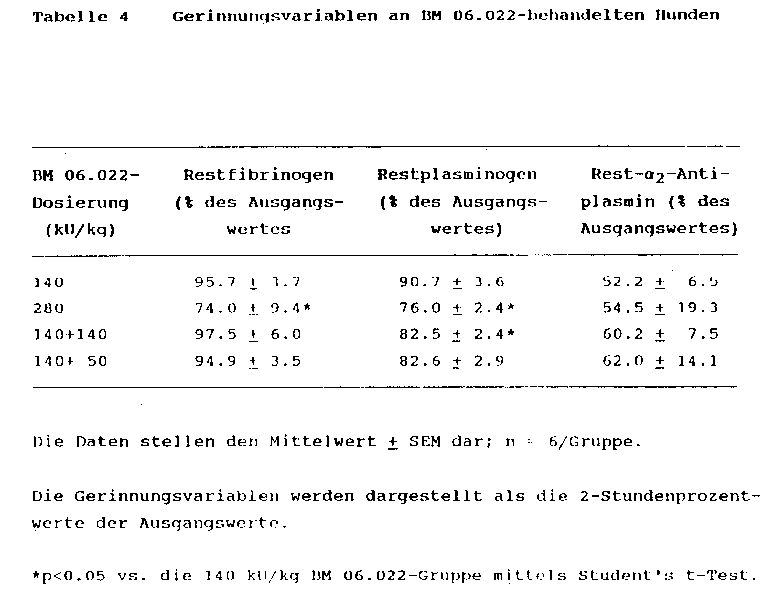

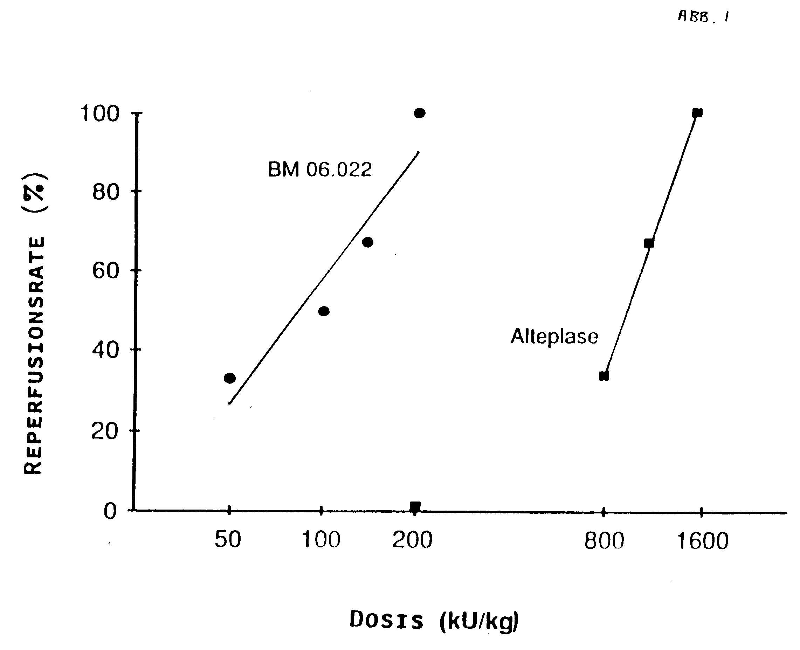

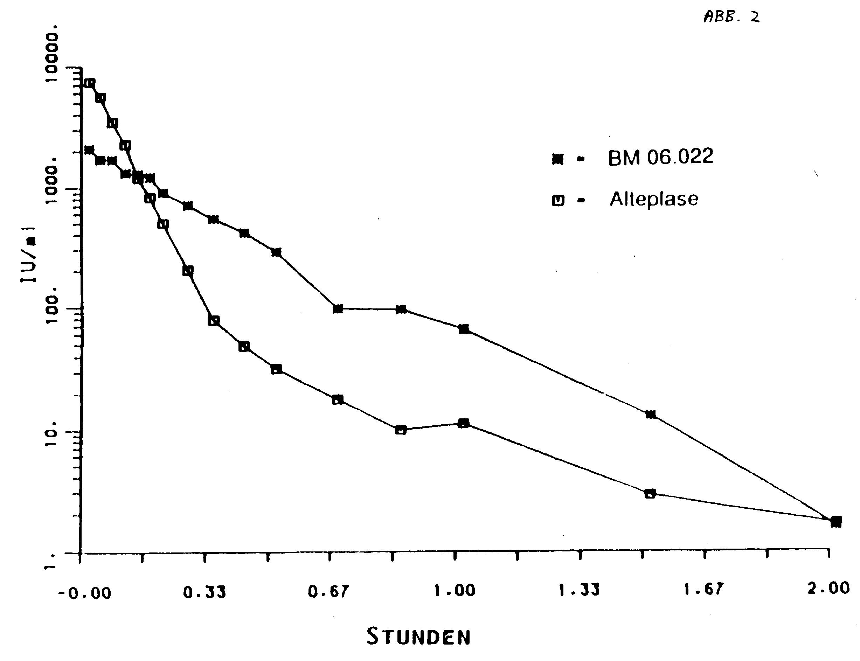

**Abbildung 1** zeigt die Dosis-Wirkungsbeziehung für die Reperfusionsrate nach Bolusinjektion von BM 06.022 oder Alteplase. Eine 100%ige Reperfusionsrate (6 von 6 Tieren) wurde mit i. v. Bolusinjektion von 200 KU/kg bei BM 06.022 erreicht. Bei der gleichen Dosis reperfundierte keines der mit Alteplase injizierten Tiere. Zur Erzielung des gleichen, maximalen Effektes war eine Injektion von 1600 KU/kg (= 2 mg/kg) Alteplase notwendig. Diese Injektionsdosis ist ungefähr doppelt so hoch wie die gegenwärtig in der Klinik als Infusion eingesetzte Dosis von Alteplase (ca. 1 mg/kg). Die höhere thrombolytische Potenz von BM 06.022, wie sie durch die Linksverschiebung der Dosiswirkungskurve veranschaulicht wird, kann durch die verbesserten pharmakokinetischen Eigenschaften von BM 06.022 (siehe Abb. 2) erklärt werden. Die Gesamtplasma-Clearance von BM 06.022 beträgt 4.4 ± 0.4 und die von Alteplase 20.4 ± 2.0 ml/min⁻¹ x kg⁻¹. Damit ist die Gesamtplasma-Clearance (ein Maß für die Entfernung einer Substanz aus dem Plasma) von BM 06.022 ca. 4,6-fach langsamer als die von Alteplase. Weiterhin wurde gefunden, daß Alteplase nach Infusion eine zwei mal höhere Reperfusionsrate als nach Einmal-Bolusinjektion der gleichen Dosis (800 KU/kg) erreichte. BM 06.022 in einer Dosis von 140 KU/kg zeigt die gleiche Reperfusionsrate (4 von 6 Tieren) nach Injektion und nach Infusion, aber die Zeit bis zur Reperfusion war nach Injektion signifikant kürzer (Tabelle 1).

Die Ergebnisse zeigen, daß Alteplase günstigere thrombolytische Erfolge nach Infusion als nach Injektion aufweist. Dieser Befund deckt sich mit der klinischen Praxis, in der Alteplase üblicherweise als Infusion verabreicht wird (Chesebro et al., Circulation 1987; 76: 142-254). Die langsamere Elimination von BM 06.022 im Vergleich mit Alteplase aus dem Plasma führte zu einer höheren thrombolytischen Potenz von BM 06.022. Dadurch ist nicht nur eine Dosisverringerung möglich, sondern es findet sich nach einmaliger i. v. Bolusinjektion überraschenderweise eine schnellere Reperfusion als nach Infusion.

### Beispiel 2

Für die Versuche in Beispiel 2 wurde das gleiche Hundemodell der Koronararterien-Thrombose wie in Beispiel 1 beschrieben benutzt. Im Gegensatz zu den Versuchen in Beispiel 1 war jedoch das Thrombusalter bei Verabreichung der Substanzen eine Stunde anstatt nur 30 Minuten. BM 06.022 wurde entweder als eine einmalige i. v. Bolusinjektion oder als eine Doppel-Bolus-Injektion jeweils über eine Minute verabreicht. Die Einmal-Bolus-Dosis betrug 140 oder 280 KU/kg. In den beiden Gruppen mit der Doppel-Bolus-Verabreichung betrug die erste Bolus-Dosis 140 KU/kg jeweils, gefolgt von dem zweiten Bolus in einer Dosis von 140 oder 50 KU/kg BM 06.022 (44 Minuten später). Jede der vier Versuchsgruppen bestand aus sechs Hunden. Zusätzliche Parameter für die Auswertung waren das Maximum des koronaren Blutflußes, das nach Reperfusion gemessen wurde, und der koronare Blutfluß bei Beendigung des Versuches drei Stunden nach Injektion. Weiterhin wurde die kumulative Patencyzeit berechnet; darunter versteht man die Summe der Zeitintervalle nach Reperfusion, in denen ein koronarer Blutfluß vorhanden war. Das Tiermodell ist so angelegt, daß typischerweise nach Reperfusion zyklische Flowvariationen mit Reokklusion auftreten. Bei Versuchsende wurde der Restthrombus entnommen und sein Feuchtgewicht gemessen.

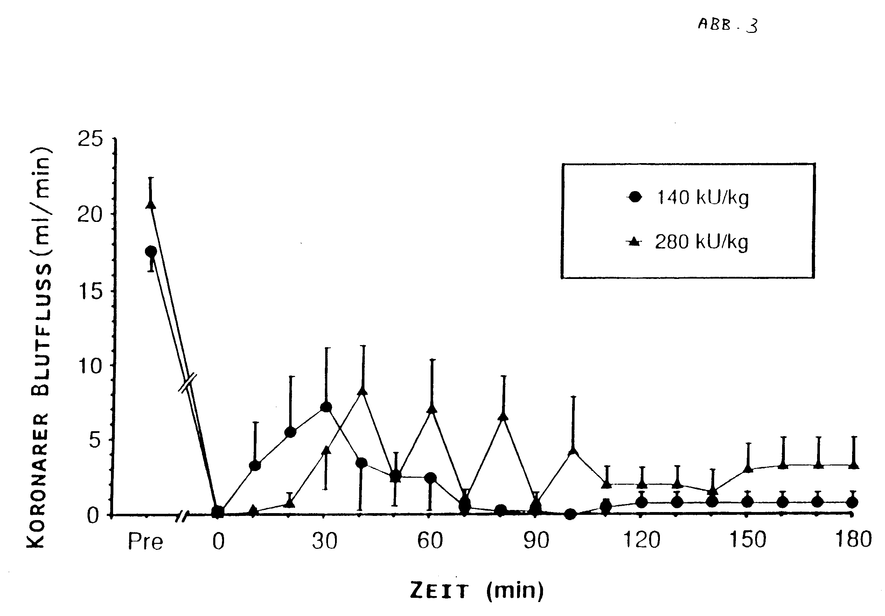

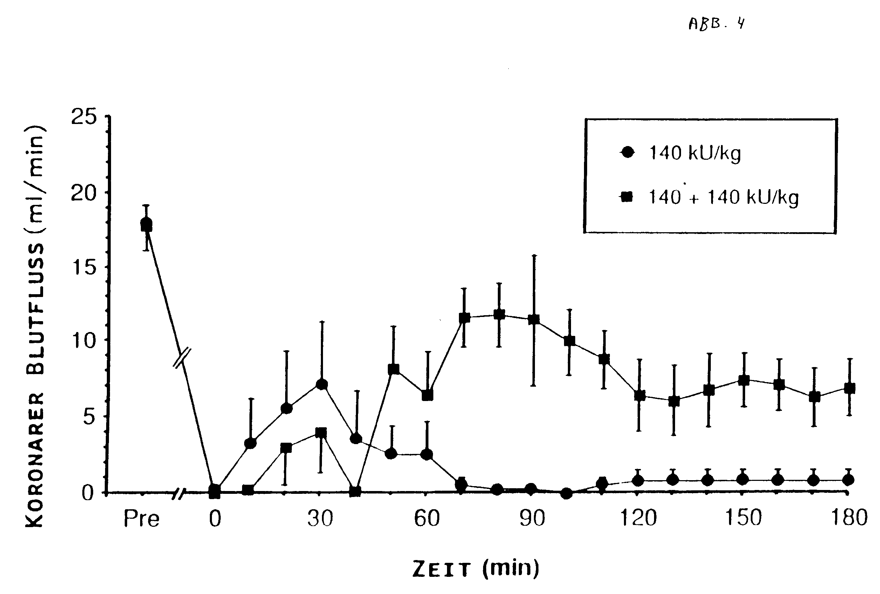

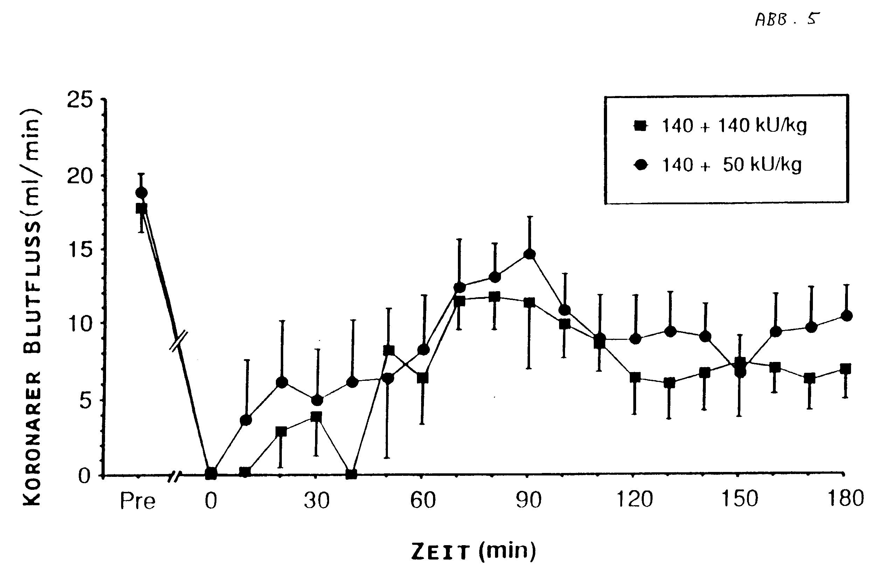

In beiden Gruppen mit Einmal-Bolus-Injektion betrug die Reperfusionsrate vier von sechs Tieren. In den Doppel-Bolus-Gruppen war sie höher und betrug sechs von sechs oder fünf von sechs Tieren (Tabelle 2). Die in Tabelle 3 dargestellten Ergebnisse für die kumulative Patencyzeit, den koronaren Blutfluß und das Restthrombusgewicht zeigen, daß eine Doppel-Bolus-Verabreichung von BM 06.022 signifikant die kumulative Patencyzeit verlängert, signifikant den koronaren Blutfluß erhöht, dieser auch bei Versuchsende signifikant erhöht geblieben ist und das Rest-Thrombusgewicht signifikant im Vergleich mit der Einmal-Bolus-Injektion von 140 KU/kg erniedrigt hat. Abbildung 3 zeigt, daß eine Erhöhung der Einmal-Bolus-Injektion von 140 auf 280 KU/kg den koronaren Blutfluß nicht deutlich verbessern kann. Es werden immer noch starke zyklische Flowvariationen beobachtet. In Abbildung 4 ist dagegen zu erkennen, daß die Doppel-Bolus-Verabreichung von 140 und 140 KU/kg BM 06.022 den koronaren Blutfluß deutlich im Vergleich mit der Einmal-Bolus-Injektion von 140 KU/kg BM 06.022 verbessert. Der gleiche Effekt auf den koronaren Blutfluß kann auch durch die Doppel-Bolus-Verabreichung von 140 und 50 KU/kg BM 06.022 erzielt werden (Abbildung 5). Wie Tabelle 4 zeigt, verhindert die Aufteilung einer Gesamtdosis von 280 KU/kg auf 140 und 140 KU/kg anstatt der Einmal-Bolus-Injektion der gleichen Gesamtdosis an BM 06.022 den signifikanten Abfall des Plasmafibrinogens.

Die Ergebnisse zeigen, daß die Verschlechterung des koronaren Blutflußes nach Reperfusion, die an diesem Modell in Simulation von Patienten mit Reokklusionsneigung intrinsisch auftritt, durch die Einmal-Bolus-Injektion selbst in der höheren Dosis nicht verhindert werden kann. Dagegen ist die Verabreichung eines Doppel-Bolus überraschenderweise in der Lage, sowohl die kumulative Patencyzeit zu verlängern als auch den Flow quantitativ zu erhöhen und bis zum Versuchsende erhöht aufrechtzuerhalten. Zusätzlich hat die Doppel-Bolus-Injektion den überraschenden Vorteil eines geringeren Abfalls des Plasmafibrinogens im Gegensatz zur Einmal-Bolus-Injektion derselben Gesamtdosis. Die Höhe des Zweitbolus in der Doppel-Bolus-Verabreichung kann dabei variabel gestaltet werden, ohne den Erfolg einzuschränken.

### Beschreibung der Abbildungen

**Abbildung 1** stellt den thrombolytischen Dosiswirkungsbereich von BM 06.022 und Alteplase nach 1-minütiger i. v. Injektion an Hunden mit Koronarthrombose dar. Die Reperfusionsrate (%) bedeutet den Prozentsatz reperfundierter Hunde innerhalb einer Dosisgruppe (n = 6 pro Dosis). Die Dosiswirkungskurven wurden mit Hilfe einer halblogarithmischen Regressionsanalyse erstellt. Die Alteplasegruppe ohne Reperfusion (bei 200 KU/kg) wurde von der Regressionsanalyse ausgeschlossen.

**Abbildung 2** zeigt den pharmakokinetischen Vergleich der Plasmakonzentrations-Zeitkurven für die Aktivität von BM 06.022 und Alteplase an anästhesierten Hunden. Eine einminütige i. v. Injektion von 140 KU/kg BM 06.022 oder 800 KU/kg (= 1 mg/kg) Alteplase wurde sechs Hunden pro Substanz verabreicht. Die Daten stellen den arithmetischen Mittelwert dar. Die Ausgangskonzentration wurde von den nachfolgend gemessenen Konzentrationen abgezogen.

**Abbildung 3** zeigt den Zeitverlauf des koronaren Blutflußes vor und nach einer einmaligen i. v. Bolusinjektion mit 140 oder 280 KU/kg BM 06.022 zum Zeitpunkt t = 0 Minuten. Die Daten sind Mittelwerte ± SEM; n = 4 reperfundierte Hunde pro Gruppe. Pre stellt den Ausgangsblutfluß dar.

**Abbildung 4** zeigt den Zeitverlauf des koronaren Blutflußes vor und nach einer Einmal i. v. Bolusinjektion von 140 KU/kg BM 06.022 (n = 4) zum Zeitpunkt t = 0 Minuten oder einer Doppel-Bolus-Injektion von 140 und 140 KU/kg BM 06.022 (n = 6) zum Zeitpunkt t = 0 und t = 44 Minuten. Die Daten sind Mittelwerte ± SEM; Pre ist der Ausgangsblutfluß.

**Abbildung 5** stellt den Zeitverlauf des koronaren Blutflußes vor und nach Doppel-Bolus-Verabreichung von 140 und 140 KU/kg (n = 6) oder 140 und 50 KU/kg BM 06.022 (n = 5) zum Zeitpunkt t = 0 und t = 44 Minuten. Die Daten stellen den Mittelwert ± SEM dar; Pre ist der Ausgangsblutfluß.

## Patentansprüche

1. Verwendung von Plasminogenaktivatoren zur Herstellung von Arzneimittlen zur Verlängerung der kumulativen Patencyzeit bei der Behandlung von thromboembolischen Erkrankungen durch Applikation eines Plasminogenaktivators in Form von zwei oder mehreren Bolusinjektionen.

2. Verwendung von Plasminogenaktivatoren nach Anspruch 1, dadurch gekennzeichnet, daß die Plasminogenaktivatoren eine gegenüber tPA mindestens um den Faktor zwei verlängerte Halbwertszeit besitzen.

3. Verwendung von Plasminogenaktivatoren nach Anspruch 1 oder 2 zur Herstellung von Arzneimitteln zur Vermeidung von Reoklusionsneigungen bei der Behandlung von Koronarverschlüssen.

4. Verwendung von Plasminogenaktivatoren nach den Ansprüchen 1 - 3, dadurch gekennzeichnet, daß die Plasminogenaktivatoren ausgewählt sind aus der Gruppe bestehend aus rekombinant hergestellten Plasminogenaktivatoren vom Typ K2P, K1K2P, D-K2P, FK2K2P, FK2P, DELTA1x, FK1K2P und tPA-Varianten.

5. Verwendung von Plasminogenaktivatoren nach den Ansprüchen 1 - 4, wobei die applizierte Menge des Plasminogenaktivators pro Bolusinjektion 3 - 50 MU beträgt.

6. Verwendung von Plasminogenaktivatoren nach Anspruch 5, wobei die erste Bolusinjektion in einer Menge von 5 - 20 MU und die zweite Bolusinjektion in einer Menge von 3 - 15 MU erfolgt.

7. Verwendung von Plasminogenaktivatoren nach den Ansprüchen 1 - 6, wobei die verabreichte Gesamtmenge des Plasminogenaktivators 15 - 40 MU beträgt.

8. Verwendung von Plasminogenaktivatoren nach den Ansprüchen 1 - 7, dadurch gekennzeichnet, daß das Zeitintervall zwischen der ersten und einer zweiten Bolusinjektion 10 - 90 Minuten beträgt.

9. Pharmazeutische Verpackungseinheit umfassend im wesentlichen eine pharmazeutische Darreichungsform eines Plasminogenaktivators in zwei oder mehreren getrennten Behältnissen.

10. Pharmazeutische Verpackungseinheit nach Anspruch 9, dadurch gekennzeichnet, daß der Plasminogenaktivator eine gegenüber tPA mindestens um den Faktor zwei verlängerte Halbwertszeit besitzt.

11. Pharmazeutische Verpackungseinheit nach Anspruch 9 oder 10, dadurch gekennzeichnet, daß die Plasminogenaktivatoren ausgewählt sind aus der Gruppe der rekombinant hergestellten Plasminogenaktivatoren vom Typ K2P, K1K2P, D-K2P, FK2K2P, FK2P, DELTA1x, FK1K2P und tPA-Varianten.

12. Pharmazeutische Verpackungseinheit nach den Ansprüchen 9 - 11, dadurch gekennzeichnet, daß die Behältnisse den Plasminogenaktivator jeweils in einer Menge von 3 - 50 MU enthalten.

13. Pharmazeutische Verpackungseinheit nach Anspruch 12, dadurch gekennzeichnet, daß das erste Behältnis den Plasminogenaktivator in einer Menge von 5 - 20 MU und das zweite Behältnis in einer Menge von 3 - 15 MU enthält.

14. Pharmazeutische Verpackungseinheit nach Anspruch 13, dadurch gekennzeichnet, daß die Gesamtmenge des Plasminogenaktivators 15 - 40 MU beträgt.

## Claims

1. Use of plasminogen activators for the production of pharmaceutical agents for extending the cumulative patency time in the treatment of thromboembolytic diseases by administering a plasminogen activator in the form of two or several bolus injections.

2. Use of plasminogen activators as claimed in claim 1, wherein the plasminogen activators have a half-life which is increased by at least a factor of two compared to tPA.

3. Use of plasminogen activators as claimed in claim 1 or 2 for the production of pharmaceutical agents for avoiding reocclusion tendencies in the treatment of coronary occlusions.

4. Use of plasminogen activators as claimed in claims 1 - 3, wherein the plasminogen activators are selected from the group consisting of recombinantly-produced plasminogen activators of type K2P, K1K2P, D-K2P, FK2K2P, FK2P, DELTA1x, FK1K2P and tPA variants.

5. Use of plasminogen activators as claimed in claims 1 - 4, wherein the amount of plasminogen activator administered per bolus injection is 3 - 50 MU.

6. Use of plasminogen activators as claimed in claim 5, wherein the first bolus injection is applied in an amount of 5 - 20 MU and the second bolus injection in an amount of 3 -15 MU.

7. Use of plasminogen activators as claimed in claims 1 - 6, wherein the total amount of administered plasminogen activator is 15 - 40 MU.

8. Use of plasminogen activators as claimed in claims 1 - 7, wherein the time period between the first and a second bolus injection is 10 - 90 minutes.

9. Pharmaceutical packaging unit comprising essentially a pharmaceutical form of administration of a plasminogen activator in two or several separate containers.

10. Pharmaceutical packaging unit as claimed in claim 9, wherein the plasminogen activator has a half-life which is increased by at least a factor of two compared to tPA.

11. Pharmaceutical packaging unit as claimed in claim 9 or 10, wherein the plasminogen activators are selected from the goup of recombinantly-produced plasminogen activators of type K2P, K1K2P, D-K2P, FK2K2P, FK2P, DELTA1x, FK1K2P and tPA variants.

12. Pharmaceutical packaging unit as claimed in claims 9 - 11, wherein the containers contain the plasminogen activator in an amount of 3 -50 MU in each case.

13. Pharmaceutical packaging unit as claimed in claim 12, wherein the first container contains the plasminogen activator in an amount of 5 - 20 MU and the second container in an amount of 3 - 15 MU.

14. Pharmaceutical packaging unit as claimed in claim 13, wherein the total amount of plasminogen activator is 15 - 40 MU.

## Revendications

1. Utilisation d'activateurs du plasminogène pour la préparation de médicaments destinés à prolonger la période de patence cumulative lors du traitement de thromboembolles par application d'un activateur du plasminogène sous la forme de deux ou plus de deux injections d'embol.

2. Utilisation d'activateurs du plasminogène selon la revendication 1, caractérisée en ce que les activateurs du plasminogène présentent une demi-période au moins deux fois plus longue que celle du tPa.

3. Utilisation d'activateurs du plasminogène selon la revendication 1 ou 2, pour la préparation de médicaments destinés à éviter les tendances à la réocclusion lors du traitement d'occlusions coronariennes.

4. Utilisation d'activateurs du plasminogène selon les revendications 1-3, caractérisée en ce que les activateurs du plasminogène sont choisis dans le groupe constitué d'activateurs du plasminogène préparés par des techniques recombinantes, de type K2P, K1K2P, D-K2P, FK2K2P, FK2P, DELTA1x, FK1K2P et des variantes du tPa.

5. Utilisation d'activateurs du plasminogène selon les revendications 1-4, dans laquelle la quantité d'activateur du plasminogène appliquée par embol injecté est de 3-50 MU.

6. Utilisation d'activateurs du plasminogène selon la revendication 5, dans laquelle la première injection d'embol comprend une quantité de 5-20 MU et la seconde injection d'embol comprend une quantité de 3-15 MU.

7. Utilisation d'activateurs du plasminogène selon les revendications 1-6, dans laquelle la quantité totale administrée d'activateur du plasminogène est de 15-40 MU.

8. Utilisation d'activateurs du plasminogène selon les revendications 1-7, caractérisée en ce que l'intervalle entre la première et la seconde injection d'embol est de 10-90 minutes.

9. Unité de conditionnement pharmaceutique comprenant essentiellement une forme galénique d'un activateur du plasminogène dans deux ou plus de deux conteneurs séparés.

10. Unité de conditionnement pharmaceutique selon la revendication 9, caractérisée en ce que l'activateur du plasminogène présente une demi-période au moins deux fois plus longue que celle du tPa.

11. Unité de conditionnement pharmaceutique selon la revendication 9 ou 10, caractérisée en ce que les activateurs du plasminogène sont choisis dans le groupe constitué d'activateurs du plasminogène préparés par des techniques recombinantes, de type K2P, K1K2P, D-K2P, FK2K2P, FK2P, DELTA1x, FK1K2P et des variantes du tPa.

12. Unité de conditionnement pharmaceutique selon les revendications 9-11, caractérisée en ce que les conteneurs de l'activateur du plasminogène contiennent chacun une quantité de 3-50 MU.

13. Unité de conditionnement pharmaceutique selon la revendication 12, caractérisée en ce que le premier conteneur contient l'activateur du plasminogène en une quantité de 5-20 MU et le second conteneur, en une quantité de 3-15 MU.

14. Unité de conditionnement pharmaceutique selon la revendication 13, caractérisée en ce que la quantité totale de l'activateur du plasminogène est de 15-40 MU.
